# FASCICULE DE BREVET EUROPEEN

(11) **EP 1 446 666 B1**
(45) Date de publication et mention de la délivrance du brevet: **13.05.2009**
(21) Numéro de dépôt: 02796851.0
(22) Date de dépôt: 20.11.2002
(51) Int. Cl.: G01N 33/543

(54) **METHODE DE DETECTION D'ANALYTE(S) A L'AIDE DE PARTICULES MAGNETIQUES COLLOIDALES**
VERFAHREN ZUM NACHWEIS VON ANALYTEN DURCH MAGNETISCHEN TEILCHEN
METHOD FOR DETECTING ANALYTE(S) USING MAGNETIC COLLOIDAL PARTICLES

(30) Priorité: 20.11.2001 FR 0115011
(43) Date de publication de la demande: 18.08.2004
(73) Titulaire: Diagnostica Stago, 92600 Asnières (FR); Bibette, Jérôme, 75005 Paris (FR)
(72) Inventeur: BIBETTE, Jérôme, F-75005 Paris (FR); BAUDRY, Jean, F-75011 Paris (FR); ROUSSEAU, Alain, F-75004 Paris (FR)
(74) Mandataire: Bernasconi, Jean Raymond
(86) Numéro de dépôt international: PCT/FR2002/003974
(87) Numéro de publication internationale: WO 2003/044532

(56) Documents cités:
- WO-A-94/09368
- WO-A-97/20214
- WO-A-98/16101
- WO-A-98/51435
- US-A- 5 512 332

## Description

La présente invention concerne une méthode utile pour détecter au moins une espèce analytique au sein d'un échantillon liquide, biologique ou synthétique, et qui implique le couplage de cette espèce avec un réactif que l'on peut apparenter à un réactif d'agglutination.

A ce jour, de nombreuses méthodes dites d'agglutination sont déjà proposées. Elles sont majoritairement utilisées pour détecter des antigènes et/ou anticorps en vue du diagnostic. Ces méthodes sont notamment appliquées pour des dosages biologiques (tests de grossesse, tests de coagulation) ou le diagnostic de maladies infectieuses. Toutefois, ces méthodes ont généralement une sensibilité limitée. En effet, le principe général de ces tests consiste à déceler la formation d'un gel de particules colloïdales qui se forme en présence de l'analyte. L'analyte doit pouvoir, dans ce type de test, accrocher deux particules colloïdales dont les surfaces sont couvertes par une molécule reconnaissant, au sens le plus large, l'analyte à doser.
La présente invention repose plus particulièrement sur l'utilisation de particules magnétiques à titre de réactifs colloïdaux d'agglutination.
Des particules magnétiques sont déjà mises en oeuvre dans des techniques de séparation biologiques ou de tests diagnostics (WO 94/09368 ; EP 180 384 ; WO 98/51435). Les séparations magnétiques s'avèrent rapides, faciles de mise en oeuvre et nécessitent un appareillage simplifié. À partir de particules magnétiques, couvertes d'une molécule reconnaissant l'analyte à capturer, on peut séparer sous l'effet des forces magnétiques, l'analyte en question d'un mélange complexe. Cette démarche est mise à profit dans le test dit ELISA par exemple (Enzyme Linked Immunoassay). Les particules magnétiques utilisées incorporent un matériau magnétique, à savoir par exemple magnétite, ferrite, oxyde de chrome ou de nickel dans une matrice, généralement de nature organique. Elles sont fonctionnalisées en surface par des groupes réactifs de type antigène, haptène ou anticorps destinés à réagir avec l'espèce active à séparer.

De manière à éviter les problèmes associés à une rémanence magnétique, un matériau superparamagnétique est généralement privilégié au matériau ferromagnétique classique. Les ferrofluides, qui possèdent une magnétisation très élevée à saturation (supérieure à 100 mT) et ne manifestent aucune rémanence magnétique en l'absence d'un champ magnétique externe sont de bons candidats pour être intégrés dans les protocoles de préparation des colloïdes superparamagnétiques.

En ce qui la concerne, la présente invention vise à tirer profit de la faculté manifestée par ces colloïdes magnétiques à adopter une organisation structurelle spécifique sous l'effet d'un champ magnétique.

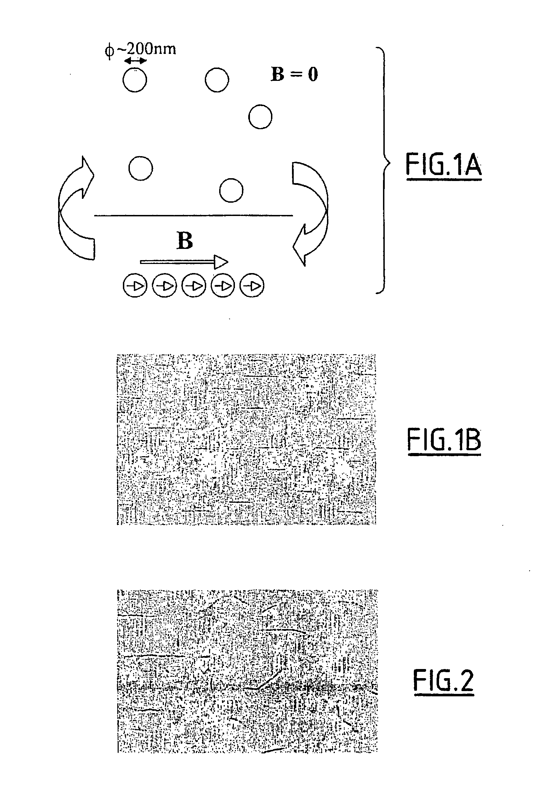

En l'occurrence, l'agglutination induite par le champ conduit, selon la fraction volumique en colloïdes, à des « chaînes » isolées ou à des amas de chaînes dénommés « clusters ». Chaînes ou Clusters seront désignés indifféremment ci-après par chaînes. On trouvera une documentation détaillée sur ces structures et leur formation dans de nombreuses publications.
En d'autres termes, en l'absence d'un champ magnétique, les particules se présentent sous une forme dispersée. En présence d'un champ magnétique, elles s'organisent pour former des chaînes de particules en solution aqueuse. Ces chaînes sont, bien entendu, réversibles et se défont rapidement à l'arrêt du champ magnétique sous l'effet de l'agitation thermique. Avantageusement, si ces particules sont suffisamment petites, et donc browniennes et polarisables, ces chaînes se forment rapidement dans l'axe du champ qui leur est appliqué et ne sont pas sensibles à la gravité. Ce phénomène est illustré schématiquement sur la figure 1A. Figure 1B montre une microphotographie des chaînes formées par de telles particules sous champ magnétique.

De manière inattendue, les inventeurs ont mis en évidence qu'il était possible d'exploiter cette faculté des particules colloïdales magnétiques à s'organiser rapidement en chaînes de particules sous l'effet d'un champ magnétique, à des fins de détection et/ou de quantification d'espèce(s) spécifique(s) dans un milieu liquide.

En l'occurrence, lorsque ces particules colloïdales magnétiques sont couvertes en surface par des ligands spécifiques et que la phase continue, dans laquelle elles sont dispersées, contient une espèce capable de réagir vis à vis d'au moins deux ligands spécifiques, le champ magnétique va catalyser l'accrochage de l'espèce considérée à deux particules distinctes et voisines dans la chaîne induite par le champ magnétique. Après annulation du champ magnétique, les particules fixées par l'espèce demeurent organisées en chaînes permanentes et indiquent ainsi la présence de l'espèce ciblée dans le milieu analysé.

Plus précisément, la présente invention vise à titre principal une méthode de détection et/ou de quantification d'au moins un analyte dans un milieu liquide, caractérisée en ce qu'elle met en oeuvre des particules colloïdales magnétiques fonctionnalisées en surface par au moins un ligand spécifique d'un analyte à détecter et/ou doser et en ce qu'elle comprend :
1. la mise en contact desdites particules avec ledit milieu,
2. l'application d'un champ magnétique audit milieu, à une intensité suffisante pour provoquer la structuration desdites particules magnétiques sous forme de chaînes,
3. le maintien de ce champ magnétique une durée suffisante pour permettre le couplage ou l'association de l'analyte considéré avec au moins deux ligands spécifiques présents respectivement sur deux particules voisines d'une chaîne,
4. l'annulation du champ magnétique, et
5. la détermination de la présence et/ou l'absence dudit analyte et, le cas échéant, de sa concentration via la présence ou l'absence de chaîne(s) permanente(s) de particules colloïdales magnétiques dans ledit milieu liquide.

Il est à noter que l'ordre dans lequel ces étapes sont énumérées correspond à un mode de réalisation privilégié de l'invention, mais qu'il est également possible dans le cadre de cette invention de les mettre en oeuvre dans un ordre différent, par exemple en activant le champ magnétique avant l'introduction du milieu à analyser.

On peut également mettre en oeuvre, dans le cadre de l'invention, une collecte des particules colloïdales magnétiques liées par l'analyte que l'on cherche à détecter, à doser ou à déplacer.
On peut également mettre en oeuvre dans le cadre de l'invention, des géométries de champ et de gradient de champ, ainsi que des géométries de « cellules d'agglutination » très diverses. Ces variations sont sujettes selon les cas à optimiser à la fois la rapidité de l'association ligand récepteur, et la détection des chaînes permanentes qui peuvent être de longueur et d'épaisseur tres variable selon les conditions. En particulier, l'invention englobe les dispositifs microfluidiques, tels que la largeur ou la hauteur des canaux soit inférieures à 100 µm. Des champs orientés perpendiculairement ou parallèlement à l'axe des canaux peuvent être utilisés.
Avantageusement, la méthode est mise en oeuvre dans des outils de diagnostic, tels que des dispositifs d'analyse. Les dispositifs d'analyse automatisés sont particulièrement préférés.
En ce qui concerne plus particulièrement la caractérisation des chaînes permanentes, elle peut avantageusement être réalisée par un simple examen microscopique du milieu analysé. Cependant, notamment en cas de mise en oeuvre dans un dispositif d'analyse automatisé, il est préférable de détecter la présence des chaînes par voie optique. On peut ainsi détecter la présence de chaînes par mesure de la diffusion de la lumière, par exemple par photométrie ou turbidimétrie. Une autre variante constitue le traitement de l'image captée par exemple par une caméra CCD. Cette variante a l'avantage de permettre une analyse fine de la taille des chaînes.Pour ces variantes de détection, on peut utiliser toute source de lumière appropriée, de préférence un laser.
Bien entendu, la formation des chaînes, assimilables aux agglutinats conventionnels, dépend d'une part de la concentration de l'analyte dans le milieu analysé et d'autre part de la concentration en particules. Schématiquement, on peut considérer que plus l'analyte est présent dans ledit milieu plus les chaînes seront longues et plus la concentration en particules est élevée, plus les chaînes seront épaisses et assimilables à des amas. Dans le cadre de la présente invention, il s'avère ainsi possible de déterminer la concentration en analyte à partir de la quantification de la densité de particules magnétiques organisées sous forme de chaîne(s) permanente(s). Cette mesure de densité peut être réalisée par des techniques conventionnelles. Pour un analyte donné, il peut être ainsi au préalable établi un étalonnage de référence.

La méthode revendiquée est particulièrement intéressante en terme de sensibilité. Elle s'avère ainsi avantageuse pour détecter des analytes à faibles concentrations dans des milieux liquides.

Comme il est montré dans l'exemple ci après, les chaînes persistent pour des concentrations en analytes, en l'occurrence en antigènes, de l'ordre de 10⁻⁹mole/l contre 10⁻⁵mole/l pour un test d'agglutination classique, mené avec le même couple ligand récepteur (Biotine streptavidine). Avantageusement, ce seuil de concentration est plus faible que le seuil de concentration nécessaire à la persistance d'agrégats ou la formation d'un gel tel qu'attendu dans un test d'agglutination classique, mené avec le même couple antigène anticorps, et en l'absence de champ magnétique.
La méthode revendiquée peut donc se substituer avantageusement à un test d'agglutination classique, et largement augmenter la sensibilité du test.

Au sens de la présente invention, le terme colloïdal signifie que les particules possèdent une taille comprise entre 5 et 10.000 nm et plus préférentiellement entre 100 et 500 nm. Toutefois, il est particulièrement intéressant de privilégier l'utilisation de particules les plus petites possibles tout en leur garantissant une susceptibilité magnétique suffisante pour autoriser une agglutination immédiate sous l'action conjuguée des forces dipolaires magnétiques et du mouvement brownien. Généralement, ce compromis est atteint pour des diamètres de particules d'au plus quelques microns et de préférence d'environ 0,1 à 0,5 microns lorsque la quantité de matériau magnétique, généralement de l'oxyde de fer encapsulé, est maximal.
Pour les raisons évoquées précédemment, on privilégie l'usage de particules colloïdales superparamagnétiques.
Il existe plusieurs technologies distinctes pour fabriquer des particules colloïdales superparamagnétiques possédant les qualités requises relatives à l'invention. Ce sont, par exemple, des techniques de co-précipitation d'un matériau polymérique naturel ou synthétique avec un ferrofluide aqueux, ou d'émulsification avec un ferrofluide en phase organique.

La technologie privilégiée pour accéder à ce type de matériau colloïdal de manière contrôlée est basée sur un principe d'émulsification suivie de protocoles de polymérisation et de greffage. Ces émulsions peuvent notamment être fabriquées par cisaillement d'un mélange constitué d'un ferrofluide organique et d'une phase aqueuse riche en tensioactif. Le ferrofluide est choisi de manière à ce que sa phase organique soit légèrement soluble dans la phase aqueuse. En l'occurrence, chaque gouttelette se transforme en un conglomérat sphérique de particules nanométriques du matériau magnétique considéré, de préférence l'oxyde de fer magnétique (maghémite) avec environ 60% en volume d'oxyde de fer au sein de chaque gouttelette. Les particules ainsi obtenues sont ensuite polymérisées en introduisant un monomère hydrophobe tel que le styrène, afin que la réaction se produise au sein des gouttelettes. L'introduction de monomères fonctionnels hydrosolubles en fin de polymérisation assure une fonctionnalisation des surfaces. L'ensemble de ces deux opérations, à savoir émulsification et polymérisation, conduit à des particules sphériques, très riches en oxyde de fer, revêtues d'une couche polymérisée et fonctionnalisée. Pour la préparation de cette émulsion, on peut notamment se reporter à la procédure publiée par J. Bibette dans J. Magn. and Magn. Mat. v. 122, p. 37 (1993) et par T Mason et J Bibette dans, Phys. Rev. Lett. 77, 3481 (1996) et dans WO 9738787 et FR 2800836.
Avantageusement, le matériau magnétique incorporé est choisi parmi les oxydes de fer, de cobalt ou des métaux divisés et stabilisés et est de préférence de la maghémite. Il est incorporé à raison de 40% en volume et de préférence de 60% dans les particules. Selon une variante préférée de l'invention, le matériau magnétique de base est un ferrofluide.
Les particules colloïdales magnétiques ainsi obtenues sont particulièrement avantageuses dans la mesure où elles sont compatibles avec l'immobilisation au niveau de leur surface d'un grand nombre de ligands, et ceci selon des méthodes industrielles conventionnelles.

Avantageusement, elles peuvent être associées à une grande variété de ligands spécifiques de manière à les transformer en réactifs utiles notamment pour des immunodosages et des tests d'agglutination tel que décrit dans la présente invention.
Les molécules dites ligands peuvent être immobilisées à la surface des particules magnétiques colloïdales par des interactions d'adsorption, covalentes et/ou de hautes affinités.
Le couplage covalent peut, par exemple, être réalisé à partir de groupements réactifs chimiques présents à la surface des particules. A titre illustratif de ces groupements, on peut plus particulièrement citer les groupements carboxyle, amino, aldéhyde et époxy. Dans le cas où l'analyte que l'on cherche à caractériser est une espèce chimique réactive, ces groupements sont eux-mêmes susceptibles d'assurer le rôle du ligand spécifique vis-à-vis de l'analyte ciblé.
Pour ce qui est de l'immobilisation relevant d'une interaction par haute affinité, elle est généralement médiée par deux partenaires d'un couple de liaison à haute affinité tels que (poly)carbohydrate/électine, biotine ou composés biotinilés/avidine ou streptavidine, récepteur de protéines et son ligand spécifique, haptène/anticorps etc...
Enfin, la fixation du ligand à la surface des particules peut être réalisée soit directement soit en utilisant des bras espaceurs encore désignés sous les termes « linker » ou « spacer »
Ces interactions covalentes ou par haute affinité permettent l'immobilisation de ligands naturels ou synthétiques, comme par exemple des peptides, des protéines, y compris les glycoprotéines, les lipoprotéines et autres structures voisines ou dérivées, sous forme libre ou complexée, des immunoglobines, des acides nucléiques comme l'ADN ou l'ARN et leurs homologues, des saccharides comme les monosaccharides ou bisaccharides, oligosaccharides et polysaccharides, des lipides, des hormones, des récepteurs, des métabolites ou autres substances biologiques.
Les analytes qui peuvent être détectés et/ou dosés par la méthode revendiquée sont de préférence des substances qui peuvent interagir spécifiquement et avec une affinité élevée avec les particules fonctionnalisées de la présente invention. Il peut ainsi s'agir d'antigènes et d'anticorps qui peuvent être déterminés par une réaction immune, ou d'acides nucléiques qui peuvent être détectés par une réaction d'hybridation, et plus généralement de toutes sortes de protéines.
De préférence, les analytes possèdent un coefficient de diffusion au sein du milieu à analyser au moins égal à celui desdites particules.

L'analyte à identifier peut sous sa forme naturelle réagir avec deux ligands distincts, à l'image par exemple des antigènes et anticorps qui offrent souvent plusieurs sites d'association.
Dans le cas contraire, il est effectué préalablement à la mise en oeuvre de la méthode revendiquée, un prétraitement du milieu à analyser afin de rendre réactif l'analyte recherché vis à vis d'au moins deux molécules d'un de ses ligands spécifiques. Ce type de prétraitement peut notamment consister en une fonctionnalisation de l'analyte par au moins une molécule d'un des partenaires des couples dits à haute affinité discutés ci-dessus. La réalisation de ce type de prétraitement relève des compétences de l'homme de l'art .

Un premier mode de réalisation de l'invention concerne la détection et/ou la quantification d'analyte(s) de type anticorps dans un échantillon liquide, par exemple des anticorps dirigés contre des pathogènes tels que virus, bactéries ou protozoaires et autres agents infectieux, anticorps dirigés contre des tumeurs, anticorps dirigés contre des allergènes, ou autoanticorps.

Un second mode de réalisation de l'invention concerne la détection et/ou la quantification d'antigènes tels que des antigènes libres comme les protéines et facteurs sanguins, tels les facteurs de la coagulation, les métabolites, hormones, médiateurs, etc., ou les antigènes liés à des supports, comme les antigènes de surface de microorganismes, les récepteurs membranaires, les antigènes des groupes sanguins et du système majeur d'histocompatibilité, etc.

La méthode revendiquée peut également être mise en oeuvre pour la détection et/ou la quantification de molécules non biologiques comme par exemple toute drogue naturelle ou artificielle, étant entendu que la drogue en question est susceptible de posséder directement une double affinité pour un ligand. Si ce n'est pas le cas un pré-traitement devra par association rendre ce type d'analyte bivalent vis à vis du ligand désigné. Cette invention peut notamment être avantageuse en chimie analytique et micro analytique.

Selon une variante particulière de l'invention, plusieurs types de particules colloïdales magnétiques peuvent être utilisés dans un même test à titre de réactifs d'agglutination. Ceci peut être avantageux dans le cas où l'analyte serait aisément réactif sur deux types de ligand.Par ailleurs, les particules colloïdales mises en oeuvre selon l'invention peuvent être porteuses d'un marqueur annexe.
Ce marquage peut être avantageux dans la mesure où il peut permettre de combiner un second mode de détection au mode de détection microscopique ou simplement d'améliorer la détection microscopique. Ce marqueur annexe peut notamment être caractérisable visuellement, optiquement, mécaniquement et/ou électriquement. Selon cette variante de l'invention, la caractérisation des chaînes peut être effectuée directement par visualisation au microscope et indirectement par une méthode optique, mécanique et/ou électrique selon la nature du marqueur retenu. A titre illustratif des marqueurs convenant à l'invention, on peut notamment citer les pigments de couleur, agents de fluorescence ou phosphorescence.
Les milieux liquides analysés peuvent être synthétiques ou naturels comme par exemple les fluides biologiques. Il peut s'agir notamment de fluides corporels comme, par exemple, le sang (total ou fractionné), le sérum, le plasma, la salive et l'urine et le liquide céphalo-rachidien utilisés sous forme diluée ou non.

En ce qui concerne le champ magnétique, il peut être créé à l'aide de différents moyens qui sont connus de l'homme de l'art, par exemple les bobines de Helmholtz, les électroaimants ou les aimants permanents.
Le champ magnétique continu appliqué est généralement compris de 1 à 500 mT, de préférence de 1 à 100 mT.

Il est généralement avantageux d'appliquer un champ magnétique essentiellement uniforme. Un moyen simple de réaliser un tel champ est de placer de part et d'autre de la zone dite d'agglutination deux pôles, respectivement Nord et Sud, d'un aimant permanent ou d'un électroaimant. Un autre moyen consiste à construire un canal d'axe essentiellement circulaire et concentrique à une bobine de Helmholtz alimentée par un générateur de courant.
Selon un mode de réalisation préféré de l'invention, on applique un champ magnétique oscillant (alternatif).
L'oscillation du champ magnétique permet d'augmenter la probabilité qu'un analyte à doser se trouve à proximité de deux ligands spécifiques présents respectivement sur deux particules. A chaque cycle de champ, les particules peuvent s'associer afin de former ou d'allonger les chaînes, sans affecter les chaînes déjà formées. Ce moyen permet donc de parfaire la réaction entre les ligands des particules colloïdales magnétiques fonctionnalisés et l'analyte à doser.
On peut ainsi doser de plus faibles concentrations d'analyte et ainsi obtenir une meilleure sensibilité du test.
Il est à noter, cependant, que certaines mises en oeuvre de l'invention peuvent requérir un champ non uniforme. En effet on pourra chercher à mettre en oeuvre à la fois un champ capable d'assurer l'agglutination des particules et un gradient de champ capable de concentrer les chaînes en un endroit précis. À la fois le champ et son gradient pourront être manipulés simultanément ou séparément. Ainsi il est possible de réaliser en premier la concentration des analytes, puis leur révélation par formation d'un amas de taille suffisante. Dans d'autres applications appartenant au cadre de l'invention il sera préférable de réaliser l'agglutination dans un canal microfluidique. On sait par exemple (E. M. Lawrence et al., Int. J. of Modern Phys. B, 8, 2765-2777, 1994) que le diamètre et l'espacement des colonnes ou chaînes riches en particules magnétiques dépendent du champ magnétique et de l'épaisseur de la cellule. Il est ainsi possible de sélectionner des conditions d'agglutinations les mieux adaptées à l'application envisagée.
Les moyens d'introduction du milieu à analyser peuvent être constitués :
- d'une ou plusieurs encoches ou « puits » dans lesquels on dépose l'échantillon, comme en électrophorèse sur gel « Electrophoresis : theory, techniques and biochemical and clinical applications, A. T. Andrews, Oxford University Press, NY 1986 »,
- ou encore d'un système de surpression ou de dépression comme en électrophorèse capillaire, voir par exemple « Capillary Electrophoresis », P. D. Grossman, J. C. Colburn eds, Academic Press, San Diego, CA, USA, 1992),
- ou encore d'un canal par lequel un filet d'échantillon est déversé en continu, comme en électrophorèse en veine liquide,
- ou encore d'un des modes d'introduction des échantillons employés en chromatographie (« Chromatographie en phase liquide et supercritique », R. Rosset, M. Caude, A. Jardy, Masson éd., Paris 1991 ; « Practical High Performance Liquid Chromatography, V. R. Meyer, John Wiley ed., Chichester, NY, USA ; « Chromatography of polymers », T. Prodver ed, ACS publ., Washington DC, 1993)
- ou encore des cuvettes ou autres contenants tels qu'utilisés conventionnellement dans les dispositifs d'analyse automatisés.

Dans sa réalisation la plus simple, l'expérience se déroule selon le protocole suivant et sur cette base il sera aisé d'imaginer de nombreuses variantes.
Des particules colloïdales ayant les propriétés décrites précédemment sont greffées par un anticorps spécifique d'un antigène à doser. Par mesure de clarté, on considère dans ce descriptif le couple ligand-récepteur streptavidine-biotine. La streptavidine étant greffée sur les particules, l'antigène à détecter, à titre d'exemple, est un petit polymère polyéthylène glycol greffé à ces deux extrémités par une molécule de biotine. Si on mélange une quantité suffisante de cet antigène avec des particules à une fraction volumique de 1% ou plus, l'agglutination sous forme de gel, comme attendu dans le test classique d'agglutination, est visible en quelques secondes, voire quelques minutes. On considère C* la concentration d'antigène telle que, en dessous de cette valeur, l'agglutination au sens classique ne soit plus décelable.

L'expérience, conforme à l'invention, consiste à mélanger des particules à une fraction volumique de l'ordre de 0.1%, avec l'antigène à une concentration C très inférieure à C* et notée C*/x, à aspirer le mélange dans un capillaire à section rectangulaire (épaisseur 20 à 50 µm) et à appliquer un champ magnétique parallèle à l'axe du capillaire. Le champ peut être appliqué grâce à deux aimants disposés de part et d'autres du capillaire. Le champ est maintenu deux minutes à titre d'exemple, avec une intensité d'au moins 500 Gauss. Le capillaire est ensuite observé avec un microscope pour déceler la présence de chaînes ou d'amas.
L'exemple du couple biotine-streptavidine permet ainsi d'établir que la détection des amas selon le protocole d'agglutination sous champ peut intervenir jusqu'à des concentrations en antigène de l'ordre de C*/1000.

La méthode revendiquée est particulièrement intéressante pour la détection et le cas échéant la quantification d'au moins un analyte dans un système microfluidique. Dans ce mode de réalisation spécifique, on peut notamment envisager de réaliser l'agglutination magnétique dans une première zone du système microfluidique et la détection dans une zone distincte. Ce mode de réalisation se prête notamment avantageusement à l'isolement des chaînes permanentes de particules, des particules n'ayant pas subi l'agglutination.
Une application particulièrement intéressante de la méthode selon l'invention constitue l'analyse multicritères, permettant le dosage simultané de plusieurs analytes dans un même test.
Selon ce mode de réalisation, on utilise plusieurs populations de particules colloïdales magnétiques fonctionnalisées. Chaque population de particules est fonctionnalisée par au moins un ligand spécifique de l'un des analytes à détecter.
L'application d'un champ magnétique oscillant conduit alors à la formation progressive de chaînes formées de particules de la même population, spécifiques pour chacun analyte.
Afin de permettre leur distinction et donc la lecture du dosage, les différentes populations de particules colloïdales magnétiques portent un marqueur annexe spécifique pour chaque analyte à détecter.

La présente invention sera mieux illustrée par les exemples qui suivent, et au regard des figures qui montrent :
Fig.1 : Schéma et microphotographie du principe de formation de lignes de particules colloïdales magnétiques sous champ magnétique.
Fig. 2 : une microphotographie d'une solution contenant des particules colloïdales magnétiques greffées et de la BSA biotinylé après arrêt du champ.
Fig. 3: un graphique montrant la longueur moyenne des lignes en fonction de la concentration en BSA biotinylé dans la solution sous champ et après arrêt du champ.
Fig. 4 A montre une microphotographie d'une solution selon l'exemple 2 pour une dilution 0% (témoin) ;
Fig. 4 B montre une microphotographie d'une solution selon l'exemple 2 pour une dilution de 0,1 % ;
Fig. 4 C montre une microphotographie d'une solution selon l'exemple 2 pour une dilution de 1% ;
Fig. 4D montre une microphotographie d'une solution selon l'exemple 2 pour une dilution de 100%.

### Exemple 1

### Détection d'un analyte à l'aide de particules magnétiques colloïdales

L'exemple illustre la détection d'un analyte modèle issu du couple de forte affinité biotine/streptavidine et vise à caractériser le seuil de sensibilité de la méthode revendiqué pour un analyte donné. L'analyte est constituée par la biotine, la streptavidine étant greffée sur les particules colloïdales.
Plus précisément, l'analyte biotinylé est composé d'un polymère Polyéthylène glycol de masse 5000, greffé à ces deux extremités par une molécule de biotine. Ces produits sont disponibles commercialement ( Shearwater USA).
Les particules superparamagnétiques colloïdales greffés par de la streptavidine sont au préalable obtenues. Les particules colloïdales sont obtenues selon la procédure publiée par J. Bibette dans J. Magn. and Magn. Mat. v. 122, p. 37 (1993) et F. Leal Calderon, T. Stora, O. Mondain-Monval, P. Poulin, and J. Bibette, Phys. Rev. Lett., 72, 2959 (1994) ou selon la procedure publié par T Mason et J Bibette dans, Phys. Rev. Lett. 77, 3481 (1996) et dans WO 9738787. La polymérisation de ces particules est obtenue selon le protocole decrit dans la demande FR 2800836. Le greffage de la streptavidine sur les sites carboxiliques est bien connu de l'homme de l'art. On pourra s'inspirer de : Molday RS, Dreyer WJ, Rembaum A, Yen SP, J Cell Biol 1975 Jan;64(1):75-88 et Staros JV, Wright RW, Swingle DM, Anal Biochem. 1986 Jul;156(1):220-2.
Le test d'agglutination sous champ consiste à mélanger les particules colloïdales magnétiques greffées par la streptavidine avec les analytes dits PEG biotine. Le mode de détection retenu est l'observation directe au microscope.
Pour une fraction volumique Φ en particules donnée, de l'ordre de 10⁻⁴ à 10⁻², on fait varier la concentration en analytes C de manière à détecter la limite de sensibilité du test.
Pour chaque essai, on aspire le mélange considéré dans un capillaire plat de 50 microns d'épaisseur, que l'on dispose entre deux aimants plats, tel que le champ produit soit supérieur à environ 50 mT et parallèle à l'axe du capillaire. Ce champ est réalisé à l'aide d'aimants plats commerciaux. Il est appliqué 2 minutes environ et le capillaire est observé au microscope en absence de champ. Le test est positif quand on peut voir sous microscope la présence de chaînes ou d'amas (voir figure 2) et négatif lorsque les particules se redispersent spontanément sous l'action de l'agitation thermique.
Il s'avère que selon ce mode de réalisation, le test demeure positif jusqu'à une concentration en analytes de l'ordre de 10⁻⁹ mol/l. A titre de comparaison, en dessous de 10⁻⁵ mol/l, il devient impossible de conduire un test d'agglutination classique.

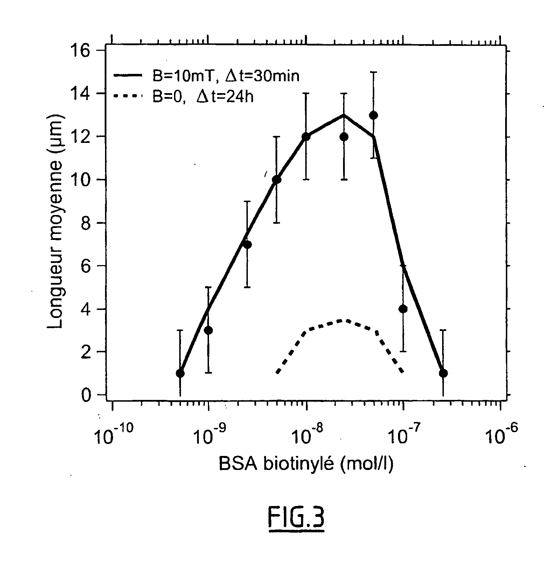

Dans une étude complémentaire, la longueur moyenne des chaînes a été déterminée en fonction de la concentration en analyte à doser (BSA biotinylé, 8 à 12 biotines/BSA). La figure 3 montre la longueur moyenne des chaînes après 30 minutes sous champ magnétique de 10 mT (ligne continue) et après 24 heures en absence de champ (ligne pointillée) en fonction de la concentration en BSA biotinylé.
On constate que l'application du champ magnétique permet la détection rapide de l'analyte pour des concentrations d'analytes pouvant descendre jusqu'à 10⁻⁹ mol/L, voire moins, ce qui représente une sensibilité supérieure à celle des tests commercialisés actuellement.
En outre, on remarque que la longueur moyenne des chaînes formées augmente en fonction de la concentration en analyte. Ainsi, la méthode de l'invention permet également détection quantitative de l'analyte à doser par le biais de la détermination de la longueur moyenne des chaînes.

### Exemple 2

### Préparation de particules colloïdales magnétigues greffées avec des immunoglobulines G anti-vWF (IgG) et dosage du facteur von Willebrand

On prépare 200 µl d'une solution colloïdale de particules à 1% en volume de particules colloïdales magnétiques (diamètre 206 nm, disponibles chez Ademtech, France) dans un tampon phosphate (20 mM, pH 7,2).

Afin d'activer les particules, la solution est additionnée de 200 µl de solution aqueuse de 1-éthyl-3-(3- dimethylaminopropyl)carbodiimide (EDAC) à 0,01 g/l et on maintient sous agitation pendant 20 minutes à 44 °C. L'excès d'EDAC est ensuite éliminé par lavage par une solution aqueuse de Tween 20 à 0,001%.
Ensuite, on procède au greffage des IgG sur les particules colloïdales en ajoutant 400 µl de solution colloïdale de particules activées dans 400 µl de solution d'IgG (1,7 µl de solution IgG à 20 mg/ml dans 398 µl d'eau). On maintient la solution sous agitation pendant 20 minutes à 44°C, puis pendant 10 minutes à température ambiante.
L'excès de solution d'IgG est ensuite éliminé par rinçage avec un tampon de saturation (tampon glycine, BSA, 0,001% de Tween 20). Les particules greffées ainsi obtenues sont ensuite séparées par filtration à travers un filtre 1,2 µm.
Les particules greffées ainsi préparées présentent un greffage d'anticorps de densité et d'orientation contrôlé. En outre, le greffage est stable, même en présence de surfactants ou d'autres protéines dans la solution. Pour le dosage du facteur von Willebrand, on utilise le plasma d'étalonnage du Liatest ® vVF (STA ® vWF Calibrator, disponible chez Diagnostica Stago) contenant 200% (2 unité internationales) de facteur von Willebrand et on procède sensiblement comme indiqué sur la notice d'utilisation de ce test, sauf à utiliser la solution colloïdale de particules magnétiques greffées.

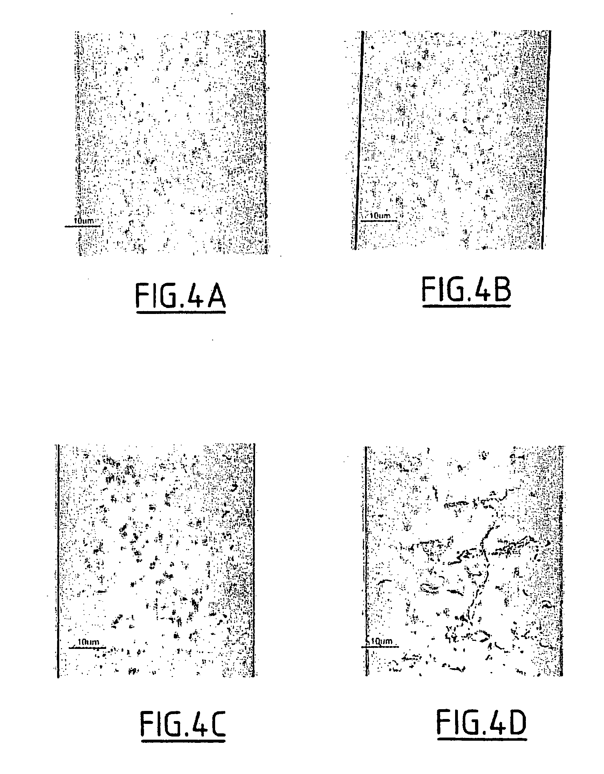

On prépare des dilutions de plasma à 100%, 1%, 0,1% de facteur von Willebrand avec du tampon Owren-Koller. A titre de témoin (0%), on utilise le tampon Owren-Koller.
Ensuite, on ajoute à 1 volume de chacune des ces dilutions 2 volumes de tampon glycine et 3 volumes de la solution colloïdale de particules magnétiques greffées.
La solution obtenue est alors placée dans un capillaire carré de 50 µm de section. L'ensemble est mis sous champ pendant 5 minutes à une intensité de 70 mT. Après arrêt du champ, l'échantillon est placé sur la platine d'un microscope optique. La figure 4 A, B, C et D montre l'apparence des échantillons sous le microscope pour les différentes dilutions étudiées. On constate notamment que la méthode décrite permet encore la distinction visuelle d'un échantillon à 0,1% du témoin (0%). Ce résultat est meilleur que le seuil de détection de 2% indiqué pour le Liatest ®.

## Revendications

1. Méthode de détection et/ou de quantification d'au moins un analyte dans un milieu liquide, **caractérisée en ce qu'**elle met en oeuvre des particules colloïdales magnétiques fonctionnalisées en surface par au moins un ligand spécifique d'un analyte à détecter et/ou doser et **en ce qu'**elle comprend :
1) la mise en contact desdites particules avec ledit milieu à analyser,
2) l'application d'un champ magnétique audit milieu, à une intensité suffisante pour provoquer la structuration desdites particules magnétiques sous forme de chaînes,
3) le maintien de ce champ magnétique une durée suffisante pour permettre le couplage ou l'association de l'analyte considérée avec au moins deux ligands spécifiques présents respectivement sur deux particules voisines consécutives d'une chaîne,
4) l'annulation du champ magnétique, et
5) la détermination de la présence et/ou l'absence dudit analyte et, le cas échéant, de sa concentration via la présence et/ou l'absence de chaîne(s) permanente(s) de particules colloïdales magnétiques.

2. Méthode selon la revendication 1, **caractérisée en ce que** lesdites particules sont des particules colloïdales superparamagnétiques.

3. Méthode selon la revendication 2, **caractérisée en ce que** les particules colloïdales superparamagnétiques sont obtenues par co-précipitation d'un polymère avec un ferrofluide aqueux ou par émulsification avec un ferrofluide en phase organique.

4. Méthode selon l'une des revendications 1 à 3, **caractérisée en ce que** les particules magnétiques colloïdales possèdent une taille comprise entre 5 et 10 000 nm et plus préférentiellement entre 100 et 500nm.

5. Méthode selon l'une des revendications 1 à 4, **caractérisée en ce que** les ligands spécifiques sont immobilisés à la surface des particules, par des interactions d'absorption, covalentes et/ou de hautes affinités.

6. Méthode selon l'une des revendications 1 à 5, **caractérisée en ce que** le ligand immobilisé est un des deux partenaires d'un couple de liaison à haute affinité.

7. Méthode selon la revendication 6, **caractérisée en ce que** le ligand est choisi parmi un des partenaires des couples (poly)carbohydrate/électine, biotine ou composés biotinilés/avidine ou streptavidine, récepteur de protéines et son ligand spécifique, et haptène/anticorps.

8. Méthode selon l'une des revendications 1 à 7, **caractérisée en ce que** le ligand immobilisé est un composé choisi parmi les peptides, protéines, y compris les glycoprotéines, les lipoprotéines, et autres structures voisines ou dérivées, sous forme libre ou complexée, des immunoglobines, des acides nucléiques comme l'ADN ou l'ARN et leurs homologues, des saccharides comme les monosaccharides ou bisaccharides, oligosaccharides et polysaccharides, des lipides, des hormones, des récepteurs, des métabolites ou autres substances biologiques.

9. Méthode selon l'une des revendications 1 à 8, **caractérisée en ce que** les analytes ciblés sont des antigènes, anticorps et des acides nucléiques et/ou protéines.

10. Méthode selon l'une des revendications 1 à 9, **caractérisée en ce que** le milieu à analyser subi au préalable un prétraitement afin de rendre réactif l'analyte recherché vis à vis d'au moins deux molécules d'un de ses ligands spécifiques.

11. Méthode selon la revendication 10, **caractérisée en ce que** le prétraitement comprend une fonctionnalisation de l'analyte par l'un des partenaires d'un couple de liaison à haute affinité.

12. Méthode selon l'une des revendications 1 à 11 pour la détection et/ou la quantification d'analyte(s) de type anticorps à des pathogènes tels que virus, bactéries ou protozoaires et autres agents infectieux, anticorps contre des tumeurs, anticorps dirigés contre des allergènes, ou autoanticorps.

13. Méthode selon l'une des revendications 1 à 11 pour la détection et/ou la quantification d'antigènes tels que des antigènes libres comme les protéines et facteurs sanguins, tels les facteurs de la coagulation, les métabolites, hormones, médiateurs, ou les antigènes liés à des supports, comme les antigènes de surface de microorganismes, les récepteurs membranaires, les antigènes des groupes sanguins et du système majeur d'histocompatibilité.

14. Méthode selon l'une des revendications 1 à 13, **caractérisée en ce que** les particules magnétiques colloïdales sont en outre porteuses d'un marqueur annexe.

15. Méthode selon la revendication 14, **caractérisée en ce que** ce marqueur est caractérisable visuellement, optiquement, mécaniquement et/ou électriquement.

16. Méthode selon la revendication 14 ou 15, **caractérisée en ce que** ce marqueur est choisi parmi les pigments de couleur et agents de fluorescence ou phosphorescence.

17. Méthode selon l'une des revendications 1 à 16, **caractérisée en ce que** l'on met en oeuvre plusieurs populations de particules colloïdales magnétiques fonctionnalisées respectivement par au moins un ligand spécifique des différents analytes à détecter.

18. Méthode selon l'une des revendications 1 à 17, **caractérisée en ce que** le champ magnétique est continu.

19. Méthode selon la revendication 18, **caractérisée en ce que** le champ magnétique continu est de 5 à 500 mT, de préférence 10 à 100 mT.

20. méthode selon l'une des revendications 1 à 19, **caractérisée en ce que** le champ magnétique est alternatif.

21. Méthode selon l'une des revendications 1 à 20, **caractérisée en ce que** la caractérisation des chaînes se fait directement par visualisation au microscope et/ou le cas échéant indirectement par toute méthode optique, mécanique, électrique.

22. Méthode selon la revendication 21, **caractérisée en ce que** la caractérisation des chaînes est réalisée par photométrie ou turbidimétrie.

23. Méthode selon la revendication 21, **caractérisée en ce que** la caractérisation des chaînes est réalisée par traitement d'image.

24. Méthode selon la revendication 23, **caractérisée en ce que** l'on utilise comme capteur d'image une caméra CCD.

25. Méthode selon l'une des revendications 21 à 24, **caractérisée en ce que** l'on utilise comme source de lumière un laser.

26. Utilisation de la méthode selon l'une des revendications 1 à 25 pour la détection et/ou la quantification d'au moins un analyte dans un système microfluidique.

## Claims

1. Method of detecting and/or quantifying at least one analyte in a liquid medium, **characterised in that** it uses magnetic colloidal particles functionalised on their surface by at least one ligand which is specific for an analyte which is to be detected and/or titrated, and **in that** it comprises:
1) contacting said particles with said medium that is to be analysed,
2) applying a magnetic field to said medium, at a sufficient intensity to cause said magnetic particles to form chain-like structures,
3) maintaining this magnetic field for a sufficient length of time to allow the coupling or combining of the analyte in question with at least two specific ligands that are present, respectively, on two consecutive adjacent particles of a chain,
4) removing the magnetic field, and
5) determining the presence and/or absence of said analyte and, if appropriate, its concentration by means of the presence and/or absence of permanent chain(s) of magnetic colloidal particles.

2. Method according to claim 1, **characterised in that** said particles are superparamagnetic colloidal particles.

3. Method according to claim 2, **characterised in that** the superparamagnetic colloidal particles are obtained by co-precipitation of a polymer with an aqueous ferrofluid or by emulsification with a ferrofluid in the organic phase.

4. Method according to one of claims 1 to 3, **characterised in that** the colloidal magnetic particles have a size of between 5 and 10,000 nm and more preferably between 100 and 500 nm.

5. Method according to one of claims 1 to 4, **characterised in that** the specific ligands are immobilised on the surface of the particles by absorptive, covalent and/or high affinity interactions.

6. Method according to one of claims 1 to 5, **characterised in that** the immobilised ligand is one of the two partners of a high affinity binding pair.

7. Method according to claim 6, **characterised in that** the ligand is selected from among one of the partners of the following pairs: (poly)carbohydrate/electin, biotin or biotinylated compounds/avidin or streptavidin, protein receptor and its specific ligand, and hapten/antibody.

8. Method according to one of claims 1 to 7, **characterised in that** the immobilised ligand is a compound selected from among peptides, proteins, including glycoproteins, lipoproteins, and other similar or derived structures, in free or complexed form, immunoglobulins, nucleic acids such as DNA or RNA and their homologues, saccharides such as monosaccharides or disaccharides, oligosaccharides and polysaccharides, lipids, hormones, receptors, metabolites or other biological substances.

9. Method according to one of claims 1 to 8, **characterised in that** the targeted analytes are antigens, antibodies and nucleic acids and/or proteins.

10. Method according to one of claims 1 to 9, **characterised in that** the medium to be analysed is subjected beforehand to a pre-treatment in order to render the analyte under investigation reactive to at least two molecules of one of its specific ligands.

11. Method according to claim 10, **characterised in that** the pre-treatment comprises functionalising the analyte with one of the partners of a high affinity binding pair.

12. Method according to one of claims 1 to 11 for detecting and/or quantifying analyte(s) of the type comprising antibodies to pathogens such as viruses, bacteria or protozoa and other infectious agents, antibodies against tumours, antibodies directed against allergens, or autoantibodies.

13. Method according to one of claims 1 to 11 for detecting and/or quantifying antigens such as free antigens against proteins and blood factors, such as clotting factors, metabolites, hormones, mediators, or antigens bound to carriers, such as surface antigens of micro-organisms, membrane receptors, antigens of blood groups and of the major histocompatibility system.

14. Method according to one of claims 1 to 13, **characterised in that** the magnetic colloidal particles also carry a linked marker.

15. Method according to claim 14, **characterised in that** the marker is visually, optically, mechanically and/or electrically detectable.

16. Method according to claim 14 or 15, **characterised in that** the marker is selected from among the colour pigments and fluorescence or phosphorescence agents.

17. Method according to one of claims 1 to 16, **characterised in that** it uses several populations of magnetic colloidal particles which have been functionalised, respectively, by at least one ligand which is specific for the various analytes which are to be detected.

18. Method according to one of claims 1 to 17, **characterised in that** the magnetic field is continuous.

19. Method according to claim 18, **characterised in that** the continuous magnetic field is from 5 to 500 mT, preferably 10 to 100 mT.

20. Method according to one of claims 1 to 19, **characterised in that** the magnetic field is alternating.

21. Method according to one of claims 1 to 20, **characterised in that** the characterisation of the chains is carried out directly by visualisation under a microscope and/or optionally indirectly by any optical, mechanical or electrical method.

22. Method according to claim 21, **characterised in that** the characterisation of the chains is carried out by photometry or turbidimetry.

23. Method according to claim 21, **characterised in that** the characterisation of the chains is carried out by image processing.

24. Method according to claim 23, **characterised in that** a CCD camera is used as the image sensor.

25. Method according to one of claims 21 to 24, **characterised in that** a laser is used as light source.

26. Use of the method according to one of claims 1 to 25 for detecting and/or quantifying at least one analyte in a microfluidic system.

## Patentansprüche

1. Verfahren zum Nachweis und/oder zur Quantifizierung zumindest eines Analyts in einem flüssigen Medium, **gekennzeichnet durch** die Verwendung von magnetischen Kolloid-Partikeln, die auf ihrer Oberfläche mit zumindest einem Liganden versehen sind, der für das zu bestimmende und/oder dosierende Analyt spezifisch ist, welches Verfahren umfaßt:
1) das In-Kontaktbringen der Partikel mit dem zu analysierenden Medium,
2) die Anwendung eines Magnetfelds auf das Medium mit einer Intensität, die ausreichend ist, um eine kettenförmige Strukturierung der magnetischen Partikel hervorzurufen,
3) die Aufrechterhaltung des Magnetfelds mit einer ausreichenden Dauer zur Ermöglichung der Verbindung oder Anlagerung des zu untersuchenden Analyts an zumindest zwei bestimmten Liganden, die jeweils auf zwei aufeinanderfolgenden benachbarten Partikeln einer Kette vorhanden sind,
4) die Beseitigung des Magnetfelds, und
5) die Bestimmung des Vorhandenseins und/oder Nichtvorhandenseins des Analyts, und ggfs. seiner Konzentration mittels des Vorhandenseins und/oder des Nichtvorhandenseins permanenter Ketten der magnetischen Kolloid-Partikel.

2. Verfahren gemäß Anspruch 1, **dadurch gekennzeichnet, dass** die Partikel superparamagnetische Kolloid-Partikel sind.

3. Verfahren gemäß Anspruch 2, **dadurch gekennzeichnet, dass** die superparamagnetischen Kolloid-Partikel erhalten werden durch Auffüllung eines Polymers mit einem wässrigen Ferrofluid oder durch Emulgieren mit einem Ferrofluid in organischer Phase.

4. Verfahren gemäß einem der Ansprüche 1 bis 3, **dadurch gekennzeichnet, dass** die magnetischen Kolloid-Partikel eine Größe zwischen 5 und 10.000 nm aufweisen, weiter vorzugsweise zwischen 100 und 500 nm.

5. Verfahren gemäß einem der Ansprüche 1 bis 4, **dadurch gekennzeichnet, dass** die spezifischen Liganden auf der Oberfläche der Partikel durch Absorptions-Wechselwirkungen, kovalente Wechselwirkungen und/oder große Anziehungen festgelegt sind.

6. Verfahren gemäß einem der Ansprüche 1 bis 5, **dadurch gekennzeichnet, dass** der festgelegte Ligand einer der zwei Partner einer Verbindung mit hoher Anziehungskraft ist.

7. Verfahren gemäß Anspruch 6, **dadurch gekennzeichnet, dass** der Ligand gewählt wird aus einem von zwei Partnern der Paare: (Poly)carbonhydrat/Elektin, Biotin oder biotinile Verbindungen/Avidin oder Streptavidin, Protein-Rezeptor und sein spezifischer Ligand, und Hapten/Antikörper.

8. Verfahren gemäß einem der Ansprüche 1 bis 7, **dadurch gekennzeichnet, dass** der festgelegte Ligand eine Verbindung ist, ausgewählt aus Peptiden, Proteinen, einschließlich Glycoproteinen, Lipoproteinen, und weiteren benachbarten oder abgeleiteten Strukturen, in freier und komplexer Form, Immunoglobinen, Nucleinsäuren wie ADN oder ARN und ihren Homologen, Sacchariden wie Monosacchariden oder Bisacchariden, Oligosacchariden und Polysacchariden, Lipiden, Hormonen, Rezeptoren, Metaboliten oder anderen biologischen Substanzen.

9. Verfahren gemäß einem der Ansprüche 1 bis 8, **dadurch gekennzeichnet, dass** die zu bestimmenden Analyten Antigene, Antikörper und Nucleinsäuren und/oder Proteine sind.

10. Verfahren gemäß einem der Ansprüche 1 bis 9, **dadurch gekennzeichnet, dass** das zu analysierende Medium zuvor einer Vorbehandlung unterzogen wird, zur Aktivierung des zu untersuchenden Analyts bezüglich zumindest zwei Molekülen von einem seiner spezifischen Liganden.

11. Verfahren gemäß Anspruch 10, **dadurch gekennzeichnet, dass** die Vorbehandlung eine Funktionalisierung des Analyts durch einen der Partner eines Verbindungspaars hoher Anziehung umfaßt.

12. Verfahren gemäß einem der Ansprüche 1 bis 11 zur Bestimmung und/oder Quantifizierung von Analyten vom Antikörper-Typ von Krankheitserregern wie Viren, Bakterien oder Einzellern oder anderen Infektionsmitteln, Antikörpern gegen Tumore, Antikörper gegen Allergene, oder Autoantikörper.

13. Verfahren gemäß einem der Ansprüche 1 bis 11 zur Bestimmung und/oder Quantifizierung von Antigenen wie freien Antigenen wie Proteinen und Blutfaktoren, wie etwa Gerinnungsfaktoren, Metaboliten, Hormonen, Mediatoren, oder trägergebundenen Antigenen wie Antigene auf der Oberfläche von Mikroorganismen, Membranrezeptoren, Antigenen der Blutgruppen und des größeren Systems der Histokompatiblität.

14. Verfahren gemäß einem der Ansprüche 1 bis 13, **dadurch gekennzeichnet, dass** die magnetischen Kolloid-Partikel unter anderem Träger eines zugehörigen Markers sind.

15. Verfahren gemäß Anspruch 14, **dadurch gekennzeichnet, dass** der Marker visuell, optisch, mechanisch und/oder elektrisch ist.

16. Verfahren gemäß Anspruch 14 oder 15, **dadurch gekennzeichnet, dass** der Marker gewählt ist aus Farbpigmenten und Fluoressenzmitteln oder Phosphoressenzmitteln.

17. Verfahren gemäß einem der Ansprüche 1 bis 16, **dadurch gekennzeichnet, dass** mehrere Gruppen magnetischer Kolloid-Partikel, die jeweils für zumindest einen spezifischen Liganden funktionalisiert sind, für verschiedene zu detektierende Analyte verwendet werden.

18. Verfahren gemäß einem der Ansprüche 1 bis 17, **dadurch gekennzeichnet, dass** das Magnetfeld kontinuierlich ist.

19. Verfahren gemäß Anspruch 18, **dadurch gekennzeichnet, dass** das kontinuierliche Magnetfeld zwischen 5 und 500 mT liegt, vorzugsweise von 10 bis 100 mT.

20. Verfahren gemäß einem der Ansprüche 1 bis 19, **dadurch gekennzeichnet, dass** das Magnetfeld ein Wechselfeld ist.

21. Verfahren gemäß einem der Ansprüche 1 bis 20, **dadurch gekennzeichnet, dass** die Charakterisierung der Ketten unmittelbar durch Visualisierung durch ein Mikroskop erfolgt und/oder ggfs. indirekt durch ein beliebiges optisches, mechanisches oder elektrisches Verfahren.

22. Verfahren gemäß Anspruch 21, **dadurch gekennzeichnet, dass** die Charakterisierung der Ketten durch Photometrie oder Turbodimetrie erfolgt.

23. Verfahren gemäß Anspruch 21, **dadurch gekennzeichnet, dass** die Charakterisierung der Ketten durch Bildverarbeitung erfolgt.

24. Verfahren gemäß Anspruch 23, **dadurch gekennzeichnet, dass** man als Bildaufnehmer eine CCD-Kamera verwendet.

25. Verfahren gemäß einem der Ansprüche 21 bis 24, **dadurch gekennzeichnet, dass** man als Lichtquelle einen Laser verwendet.

26. Verwendung des Verfahrens gemäß einem der Ansprüche 1 bis 25 zur Bestimmung und/oder Quantifizierung zumindest eines Analyts in einem Mikrofluid-System.
